# EUROPÄISCHE PATENTSCHRIFT

(11) **EP 2 326 364 B1**
(45) Veröffentlichungstag und Bekanntmachung des Hinweises auf die Patenterteilung: **08.06.2016**
(21) Anmeldenummer: 09778320.3
(22) Anmeldetag: 03.09.2009
(51) Int. Cl.: B01D 61/18, B01D 63/02, B01D 69/02, B01D 71/26, B01D 53/22, A61M 1/16, B01D 63/08, C12M 1/00

(54) **GASTRANSFERVORRICHTUNG**
GAS TRANSFER DEVICE
DISPOSITIF DE TRANSFERT DE GAZ

(30) Priorität: 03.09.2008 DE 102008045621
(43) Veröffentlichungstag der Anmeldung: 01.06.2011
(73) Patentinhaber: Novalung GmbH, 74076 Heilbronn (DE)
(72) Erfinder: MAURER, Andreas, 72072 Tübingen (DE)
(74) Vertreter: Graf von Stosch, Andreas
(86) Internationale Anmeldenummer: PCT/EP2009/006403
(87) Internationale Veröffentlichungsnummer: WO 2010/025926

(56) Entgegenhaltungen:
- EP-A2- 1 847 594
- WO-A1-02/076529
- US-A- 4 111 659

## Beschreibung

### Hintergrund der Erfindung

Die Erfindung betrifft eine Gastransfervorrichtung mit einer spezifisch strukturierten Membran, wobei die Membran durch ihre Strukturierung einen besonders effektiven Gasaustausch ermöglicht, insbesondere zwischen einer flüssigen Phase und einer gasförmigen Phase.

Gastransfervorrichtungen kommen in vielen Gebieten der Technik zum Einsatz. Dabei handelt es sich entweder um Begasungs- bzw. Entgasungsvorrichtungen, bei denen ein oder mehrere Gase von einem Medium in ein anderes übertreten, oder um Gasaustauschvorrichtungen, die den gegenseitigen Austausch eines oder mehrerer Gase zwischen zwei Medien ermöglichen. Gastransfervorrichtungen finden beispielsweise in der chemischen Verfahrenstechnik Anwendung. Sie dienen dort zur Zufuhr von Gasen für Gas/Flüssig- oder Gas/Feststoff-Reaktionen. jedoch können sie auch zur Gastrennung bzw. Gasaufreinigung eingesetzt werden, indem nicht ein Gas eingeleitet wird, sondern Gas aus einem Gasgemisch oder einem anderen Reaktionsgemisch abgezogen wird.

Daneben finden Gastransfervorrichtungen ebenfalls in der Biotechnologie und der Medizin Anwendung. Der wichtigste Einsatzort in der Biotechnologie ist deren Verwendung in Kultivierungsreaktoren. Mittels Gastransfervorrichtungen werden hier Zellkulturen spezifisch und kontrolliert mit den für die bestimmte Kultur notwendigen Gasen versorgt bzw. Ausscheidungsgase werden aus dem Nährmedium entfernt. Auch in der Medizin kommen Gastransfervorrichtungen zum Einsatz. Der wichtigste Einsatzzweck ist hier die Anreicherung des Blutes mit Sauerstoff bei gleichzeitiger Entfernung von Kohlenstoffdioxid aus dem Blut. Solche Maßnahmen sind bspw. bei diversen Operationen und bei der Behandlung von verschiedenen Lungenerkrankungen notwendig.

Lungenerkrankungen stehen mit 9 Mio. Todesfällen pro Jahr an dritter Stelle der Todesursachenstatistik der WHO. Die derzeit einzige langfristig effektive Therapieoption für Patienten mit endgradiger funktioneller Lungenerkrankung stellt die Lungentransplantation dar. Eine andere medizinische Lösung, um dauerhaft die Funktion der Lunge zu ersetzen, existiert hingegen nicht. Deshalb besteht insbesondere ein signifikantes Bedürfnis nach dauerhaften, künstlichen Lungenersatzverfahren, welche bei Patienten mit chronischen Lungenerkrankungen, die nicht für eine Lungentransplantation in Betracht kommen, angewendet werden können. Außerdem besteht ebenfalls Bedarf nach Lungenersatzvorrichtungen, die bei Patienten eingesetzt werden, die auf eine Lungentransplantation warten. Die Wartezeiten sind derzeit so lang, dass ca. 80% der Patienten vor der medizinisch indizierten Lungentransplantation versterben. Geeignete, über längere Zeiträume einsetzbare Lungenersatzvorrichtungen könnten hier Abhilfe schaffen.

Bereits in den 1950er Jahren wurden Begasungsvorrichtungen für solche Zwecke entwickelt. Diese sogenannten Oxygenatoren, d.h. Sauerstoff-Begasungsvorrichtungen, haben bis heute eine fortwährende Entwicklung durchlaufen und werden noch heute in ihrer Funktionalität weiter verbessert.

Der Prototyp eines solchen Oxygenators war ein Filmoxygenator, bei dem von einer Rollenpumpe gefördertes Blut über Siebe in fast reiner Sauerstoffumgebung mit Sauerstoff angereichert wurde. Der großflächige direkte Kontakt mit Sauerstoff führte jedoch zur Denaturierung von Plasmaproteinen - ein entscheidender Nachteil beim Einsatz des Filmoxygenators.

Nachfolgend wurde der sog. "Bubble"(Blasen)-Oxygenator entwickelt. Dabei wurde Blut in einer Blutsäule mit Gasbläschen angereichert. Durch Variieren des Gasflusses wird die Sättigungsleistung eingestellt. Der Gasaustausch findet dabei direkt an der Oberfläche der Gasbläschen statt. Das größte Problem des Bubble-Oxygenators war bzw. ist die bei dieser Sauerstoffanreicherung auftretende Aufschäumung des Blutes, die im Körper zu Mikroembolien führen kann. Nachfolgende Entschäumungsverfahren sind daher notwendig, was dieses Verfahren aufwendig und teuer macht. Beispiele für Oxygenatoren der Bubble-Art sind u.a. in DE 22 08 868, DE 23 14 644, DE 23 32 445 und DE 30 01 018 beschrieben.

Kurz nach der Entwicklung des Bubble-Oxygenators erfolgte bereits 1956 erstmalig der Einsatz eines Membranoxygenators. Beim Membranoxygenator ist die Gasphase von der Blutphase durch eine Membran getrennt. Der Gasaustausch findet an der gasdurchlässigen Membran aufgrund von Partialdruckdifferenzen der beteiligten Gase vorwiegend über Diffusion statt. Die Membranen können dabei als Flachmembranen oder als Kapillar- bzw. Fasermembranen ausgestaltet sein. Zwei Typen von Membranoxygenatoren des jüngeren Standes der Technik sind beispielsweise in US 5,137,531 und US 6,682,698 beschrieben. Ein genereller Nachteil der nach dem Diffusionsprinzip arbeitenden Membranoxygenatoren ist jedoch, dass große Membranflächen bereitgestellt werden müssen, um einen effektiven Stoffaustausch zwischen Blut und Sauerstoff in einer bestimmten Zeit zu erreichen. Die Diffusion durch die Membran kann dabei durch Erhöhung des Sauerstoffdrucks bzw. durch die Änderung der Strömungseigenschaften des Blutes beeinflusst werden. Es ist jedoch grundsätzlich recht schwierig, einen Kompromiss zwischen potentieller Blutschädigung, Thromboseneigung und effektivem Gasaustausch zu schließen.

Bei den heute überwiegend verwendeten Fasermembran-Oxygenatoren ist durch die große Gesamtoberfläche der Membran zwar eine recht gute Diffusion einstellbar, nachteilig wirkt sich für diesen Oxygenator aber die kostspielige und aufwendige Herstellung der Fasern aus. Ein weiterer Nachteil der zurzeit verwendeten Oxygenatoren ist die nur sehr kurze Haltbarkeitsdauer. So kann ein Oxygenator des Standes der Technik nur für einige Tage, höchstens bis zu einem Monat, eingesetzt werden. Eine Langzeitanwendung, wie sie insbesondere bei Patienten mit chronischem Lungenversagen wünschenswert wäre, kann gleichwohl nicht befriedigend realisiert werden.

Die beispielhaft für die in der Medizin verwendeten Oxygenatoren genannten Nachteile treten ebenfalls bei Gastransfervorrichtungen auf, welche in der Chemie und Biotechnologie verfahrenstechnisch zum Einsatz kommen.

Aus der WO 02/076529 A1 ist eine Vorrichtung zum Prozessieren von Blut bekannt, die eine erste Platte aufweist, die mit einer Anzahl von ersten Kanälen in einer ersten Richtung zum Transportieren eines Fluids, wie Blut, versehen ist. Die Vorrichtung umfasst ferner eine zweite Platte, die in der Nähe der ersten Platte angeordnet ist und die mit einer Anzahl von zweiten Kanälen in einer zweiten Richtung für den Transport von einem oder mehreren Gasen oder eines Gemisches aus gasförmigen Medien versehen ist, wobei zwischen den ersten und den zweiten Kanälen eine gasdurchlässige Membran angeordnet ist.

Aus der EP 1 847 594 A2 ist ein künstliches Lungensystem bekannt, bei dem eine Gasaustauschmembran eine Blutseite von einer Luftseite trennt, wobei die Gasaustauschmembran auf der Blutseite und der Luftseite jeweils eine Fremdoberfläche aufweist, die auf der Blutseite und/oder auf der Luftseite mit biologischen Zellen besiedelt ist. Das künstliche Lungensystem kann zur Herstellung eines extrakorporalen oder implantierbaren Lungenunterstützungssystems oder zur Herstellung eines Lungenmodellsystems für die Untersuchung von Atemwegsbelastungen verwendet werden.

### Beschreibung der Erfindung

Der vorliegenden Erfindung liegt daher die Aufgabe zugrunde, verbesserte Gastransfervorrichtungen bereitzustellen, welche in der Chemie, Biotechnologie und Medizin, insbesondere zur Begasung bzw. zum Gasaustausch im Blut, Verwendung finden können. Insbesondere soll mit der folgenden Erfindung eine Gastransfervorrichtung mit verbessertem Gastransfer durch die Membran und mit erhöhter Haltbarkeitsdauer bereitgestellt werden.

Diese Aufgabe wird erfindungsgemäß durch die gemäß Hauptanspruch genannte Gastransfervorrichtung gelöst, die mindestens zwei Kammern und mindestens eine gasdurchlässige und flüssigkeitsundurchlässige Membran umfasst, wobei die Kammern durch die Membran(en) voneinander getrennt sind und wobei die Membran(en) auf mindestens einer Seite strukturiert ist/sind und durch diese Strukturierung bzw. diese Struktur Kanäle und/oder Verästelungen auf der Membran gebildet werden, deren sich gegenüberliegende Wände eine Beabstandung von ≤ 500 µm, bevorzugt ≤ 250 µm, und weiter bevorzugt ≤ 150 µm aufweisen, und der Anteil der Membranoberfläche, der Kanäle und/oder Verästelungen mit dieser Beabstandung aufweist, mindestens 50 % der Gesamtoberfläche der Membran ausmacht, wobei die Membran auf beiden Seiten strukturiert ist, und auf mindestens einer Seite der Membran Verästelungen aufweist, die durch Noppen erzeugt sind, die derart angeordnet sind, dass sie als Hindernisse in der Flussrichtung stehen und eine Ablenkung und Aufspaltung der Strömung durch die jeweils zwei Kammern verursachen.

Die erfindungsgemäße Gastransfervorrichtung zeichnet sich durch eine besonders vorteilhaft strukturierte, mit Gasaustauschkanälen und/oder Verästelungen versehene Membran aus. In diesem Zusammenhang sind "Verästelungen" in Bezug auf die Berührungsflächen und die daraus entstehenden Wege für eine flüssige oder eine gasförmige Phase auf der Seite der Membran zu verstehen.

In einer bevorzugten Ausführungsform der Erfindung ist mindestens eine, und bevorzugt jede, der mindesten zwei Kammern der Gastransfervorrichtung als Durchflusskammer gestaltet. Die sogenannten "Verästelungen" in der Strukturierung bzw. Struktur der Membran stellen daher verästelte oder verwinkelte Wege für den Fluss der flüssigen oder gasförmigen Phase über die jeweilige Seite der Membran bzw. durch die Kammer dar. Die Verästelungen können z.B. durch Stellen in der Membranstruktur entstehen, die als Hindernisse in der Flussrichtung stehen und eine Ablenkung und/oder eine Spaltung bzw. Aufspaltung der Strömung durch die jeweilige Kammer verursachen. In diesem Sinne ist die Membran auf beiden Seiten z.B. derart strukturiert, dass die Wände der Struktur aus einer Ebene der Membran herausragen bzw. hervorstehen und/oder in die jeweilige angrenzende Kammern hineinragen bzw. hineinreich. Diese Wände können bspw. von der flüssigen oder gasförmigen Phase in der Kammer umflossen sein. Diese Wände der Struktur, mit der die mindestens eine Seite der Membran versehen ist, bilden bspw. Vorsprünge, welche geometrische Formen, wie z.B. Quader, Rauten, Zylinder oder Pfosten, haben können und zu einer Verästelung der Membranseite führen. Ebenso können diese Wände Kanäle (z.B. verästelte Kanäle) bilden, welche die flüssige oder gasförmige Phase in der Kammer über die Membranseite führen.

Die erfindungsgemäße Strukturierung bzw. Struktur der Membran ermöglicht einen verbesserten Stofftransport (durch insbesondere verbesserte Diffusionseigenschaften) durch die Membran, ohne dass die Membran allerdings, wie im Stand der Technik vorgesehen, großflächig dimensioniert sein müsste. Dies beruht einerseits darauf, dass durch die Strukturierung eine größere Oberfläche geschaffen wird, die sich positiv auf den Gasübertritt auswirkt. Andererseits weisen die Wände der durch die Strukturierung entstandenen Kanäle und/oder Verästelungen auf der Membran eine kleine Beabstandung auf, wodurch die Diffusion durch die Membran beschleunigt wird. Die geringe Beabstandung der Kanäle und/oder Verästelungen sorgt dabei für eine deutliche Herabsetzung des Flusswiderstandes. Dies ist insbesondere bei Flüssigkeiten wie beispielsweise biologischen Flüssigkeiten, von Bedeutung. Bei der Verwendung von Blut führt der sog. Fareus-Lindquist-Effekt dazu, dass sich an den Wänden ein Film aus Blutplasma bildet, auf dem die Zellen durch die erfindungsgemäß strukturierten Kanäle und/oder Verästelungen gleiten.

Erfindungsgemäß ist die mindestens eine gasdurchlässige und flüssigkeitsundurchlässige Membran der erfindungsgemäßen Gastransfervorrightung, die die Kammern voneinander trennt, auf beiden Seiten strukturiert. Beim Einsatz von Flüssigkeiten in der/den Kammer(n) ist vorzugsweise die der Flüssigkeit zugewandte Membranseite strukturiert. Insbesondere bei Flüssigkeiten werden die positiven Eigenschaften (z.B. die Herabsetzung des Flusswiderstandes) einer strukturierten Membran auf den Gasübertritt deutlich.

Erfindungsgemäß ist mindestens eine und bevorzugt jede der Kammern von der Membran bzw. von der Membranstruktur mindestens teileweise gebildet. Mit anderen Worten ist jede Kammer in einer bevorzugten Form der Erfindung mindestens teilweise von der Membran abgegrenzt bzw. umschlossen.

In einer bevorzugten Ausführungsform der Erfindung umfassen die mindestens zwei Kammern eine erste Kammer zur Aufnahme bzw. zum Durchfluss einer biologischen Flüssigkeit und eine zweite Kammer zur Aufnahme bzw. zum Durchfluss eines Gases. Somit dienen die durch die Strukturierung gebildeten Kanäle und/oder Verästelungen einem Transfer von Gasmolekülen zwischen dem Gas und der biologischen Flüssigkeit.

In einer weiter bevorzugten Ausführungsform der erfindungsgemäße Gastransfervorrichtung ist die erste Kammer in mehreren Kammern unterteilt, sodass die Vorrichtung mehrere erste Kammern umfasst, die von der zweiten Kammer durch eine gasdurchlässige und flüssigkeitsundurchlässige Membran getrennt sind. Ebenso ist die zweite Kammer vorzugsweise in mehreren Kammern unterteilt ist, sodass die Vorrichtung mehrere zweite Kammern umfasst, die von der bzw. den ersten Kammer(n) durch eine gasdurchlässige und flüssigkeitsundurchlässige Membran getrennt sind. Die mehreren ersten Kammern können in einer oder mehreren Reihen nebeneinander, und/oder in mehreren Lagen übereinander angeordnet sind. In ähnlicher Weise können die mehreren zweiten Kammern in einer oder mehreren Reihen nebeneinander und/oder in mehreren Lagen übereinander angeordnet sind. Ferner kann sich eine längliche Ausrichtung der ersten Kammer(n) bspw. quer und bevorzugt rechtwinkelig zu einer länglichen Ausrichtung der zweiten Kammer(n) erstrecken. jede der ersten Kammern ist damit vorzugsweise für einen Durchfluss von einem Einlass zu einem Auslass in einer Richtung gegen oder quer zur allgemeinen Flussrichtung der zweiten Kammer(n) ausgebildet. In ähnlicher Weise ist jede der zweiten Kammern vorzugsweise für einen Durchfluss von einem Einlass zu einem Auslass in einer Richtung gegen oder quer zur allgemeinen Flussrichtung der ersten Kammer(n) ausgebildet.

Erfindungsgemäß kann die Membran aus einem organischen oder einem anorganischen Material bestehen oder ein solches umfassen. Zu anorganischen Membranmaterialien zählen Glas, Keramik (z.B. Aluminiumoxid, Titandioxid oder Zirkoniumoxid), Metall, Silikon oder Kohlenstoff. Zu organischen Membranmaterialien gehören insbesondere Polymermaterialien wie etwa Polyacrylamide, Polyacrylnitrile, Polyamide, Polybenzimidazole, Polybutadiene, Polycarbonate, Polydimethylsiloxane, Polyethersulfone, Polyetherimide, Polyolefine, Polyethylenterephtalate, Polymethylmethacrylat, Polymethylpenten, Polyphenylenoxid, Polystyrol, Polysulfone, Polyvinylalkohol, Polyvinylchlorid, Polyvinylidenfluorid, andere halogenierte Kohlenwasserstoffe und Zellulose und zyklische Olefincopolymere (COC). Organische Membranmaterialien können entweder das reine Polymer, ein Polymerkomposit (d.h. Mischung verschiedener Polymere oder Copolymere) oder Polymerschichtungen (d.h. Polymerlaminate) umfassen.

In einer bevorzugten Ausführungsform der vorliegenden Erfindung ist das Membranmaterial ein organisches Material, ausgewählt aus den oben genannten Materialien. Noch stärker bevorzugt ist das Membranmaterial ein Polymer, ein Polymerkomposit oder eine Polymerschichtung der genannten Materialien.

In einer weiteren bevorzugten Ausführungsform der Erfindung ist das Polymer ein Polyolefin, wie beispielsweise Polyethylen, Polypropylen, Polybutadien oder Polypenten oder Methylvarianten davon. Am stärksten bevorzugt besteht oder umfasst das Membranmaterial Polymethylpenten. PDMS kann aber auch verwendet werden.

Ein in diesem Zusammenhang besonders geeignetes Membranmaterial stellt das unter der Produktbezeichnung TPX™ vertriebene Methylpentenpolymer der Firma Mitsui Chemicals dar. Neben vielen vorteilhaften Eigenschaften (beispielsweise gute Biokompatibilität) zeichnet sich TPX™ durch eine besonders hohe Sauerstoffpermeabilität aus (z.B. 47.000 ml/m² pro Stunde bei 24 atm, 25°C, 90% relativer Feuchte (RH), und 25 µm Membranstärke; i.e. eine Sauerstoffpermeabilität von 47.000 ml/m² je 24 Stunden (24 h) je bar Druckunterschied (d.h. 47.000 ml/m².24h.bar) bei 25°C, 90% relativer Feuchte (RH) und einer Membranstärke von 25 µm). Zusätzlich besitzt TPX™ eine geringe Feuchtigkeitsdurchlässigkeit von 110 g Wasser je m², innerhalb von 24 h bei 40°C und 90% relativer Feuchte (RH) und eine geringe Permeabilität für Stickstoff. Deshalb ist TPX™ als Membranmaterial für die erfindungsgemäße Gastransfervorrichtung besonders geeignet, insbesondere für Anwendungen mit Bezug zum Sauerstofftransport, bspw. zur Anreicherung von Blut mit Sauerstoff.

Abhängig vom gewählten Membranmaterial sind verschiedene Möglichkeiten zur Herstellung strukturierter Membranen bzw. zur Strukturierung bereits bestehender Membranen bekannt. Das Standardverfahren zur Herstellung von sowohl organischen als auch anorganischen Membranen ist das Sintern, wobei die Erzeugung des Sintermaterials wiederum durch eine Vielzahl von Verfahren, wie Pressverfahren, Extrudieren, Filmgießen, Sedimentationsverfahren und Sol-Gel-Verfahren erfolgen kann. Für organische Membranen gibt es eine Reihe weiterer Herstellungsverfahren, wie z.B. das Recken (Strecken eines Polymers rechtwinklig zur Extrusionsrichtung), das Kernspurverfahren (radioaktive Bestrahlung mit anschließendem Ätzprozess), das Phaseninversionsverfahren (Fällungsreaktion) und das Schäumen (Porenbildung durch CO₂-Expansion). Meist ist der industrielle Aufwand für die Herstellung von organischen Membranen geringer als der für anorganische Membranen. Die verwendete Membran kann nach einem beliebigen der genannten oder auch nach anderen, aus dem Stand der Technik bekannten Verfahren hergestellt bzw. strukturiert werden.

Vorzugsweise wird die Membran durch lithographische Verfahren, insbesondere die LIGA-Technik strukturiert. LIGA (Abk. für die Verfahrensschritte Lithographie, Galvanik und Abformung) bezeichnet ein Verfahren, welches auf einer Kombination von Tiefenlithographie, Galvanik und Mikroabformung basiert. Das LIGA-Verfahren wurde Anfang der 80er Jahre im Rahmen der Entwicklung des Trenndüsenverfahrens zur Urananreicherung entwickelt, um extrem kleine Trenndüsen herstellen zu können (E.W. Becker et al., Naturwissenschaften 69, 520-523 (1982)). Das Verfahren ermöglicht die Herstellung von Mikrostrukturen mit kleinsten Abmessungen aus verschiedenen Materialien wie Kunststoff, Metall oder Keramik (E.W. Becker et al., Microelectronic Engineering, 4, 35-56 (1986)). Insbesondere wird die LIGA-Technologie auch im Bereich der Mikrosystemtechnik verwendet.

Abhängig von der beim Lithographie-Verfahren verwendeten Strahlung wird zwischen Röntgen-LIGA und UV-LIGA unterschieden. Das LIGA-Verfahren besteht typischerweise aus folgenden Schritten, die nacheinander durchgeführt werden: Zunächst wird eine bis zu 1-mm-starke Röntgen- bzw. UV-empfindliche Kunststoffschicht (z.B. PMMA) auf eine Grundplatte mit elektrisch leitender Deckschicht aufgebracht. Danach erfolgt die lithographische Tiefenstrukturierung, indem ein Resist belichtet wird. Die belichteten Bereiche werden nachfolgend mit einem geeigneten Entwickler herausgelöst, wobei eine Negativform der Metallstruktur bestehen bleibt, die in der Galvanik erzeugt werden soll. In einem nachfolgenden galvanischen Verfahren wird ein Metall auf dem Substrat in den Bereichen abgeschieden, in denen der Resist beim Entwickeln entfernt worden ist. Der Resist wird nachfolgend herausgelöst, wobei die abgeschiedene Metallstruktur zurückbleibt. Diese Metallstruktur dient als ein Abformwerkzeug für Abformungstechniken wie Heißprägen und Spritzgießen, mit dem insbesondere organische Membranen (beispielsweise aus Kunststoff) erzeugt werden können. Neben dem Heißprägen und Spritzgießen können auch ein Rolle-zu-Rolle-Verfahren oder Vakuumtiefziehen zur Abformung eingesetzt werden. Weitere Beispiele der Technik zum Formenbau sind Mikropräzisionsfräsen und Ultrapräzisionsfräsen.

Die in der Gastransfervorrichtung verwendete Membran ist erfindungsgemäß auf mindestens einer Seite strukturiert, bevorzugt ist die Strukturierung erhältlich unter Einsatz von Mikrospritzguss oder Heißprägeverfahren. In einer weiteren Ausführungsform ist die Membran auf beiden Seiten strukturiert, ebenfalls bevorzugt erhältlich mit Hilfe von Spritzguss- oder Heißprägeverfahren.

Das LIGA-Verfahren stellt eine bisher zur Herstellung von strukturierten Membranmaterialien noch nicht verwendete Technik dar, wobei dadurch Produkte kostengünstig und in hohen Stückzahlen gefertigt werden können. Deshalb betrifft die vorliegende Erfindung ebenfalls ein Verfahren zur Herstellung strukturierter Membranen durch das LIGA-Verfahren und die dadurch erhaltenen Membranen.

Die Strukturierung der Membranen erfolgt im Mikrometer-Bereich. Erfindungsgemäß werden durch die Strukturierung Kanäle und/oder Verästelungen auf der Membran gebildet, deren Wände eine Beabstandung von ≤ 500 µm, bevorzugt ≤ 350 µm, weiter bevorzugt ≤ 150 µm, stärker bevorzugt ≤ 100 µm, und noch stärker bevorzugt ≤ 80 µm aufweisen. Die Abmessung bzw. die Höhe dieser Wände, insbesondere aus der Ebene der Membran, ist typischerweise in der selben Größenordnung wie die Beabstandung und liegt vorzugsweise im Bereich von 10-350 µm, weiter bevorzugt im Bereich von 10-200 µm, und noch weiter bevorzugt im Bereich von 10-100 µm. Erfindungsgemäß macht der Anteil der Membranoberfläche, der Kanäle und/oder Verästelungen aufweist, ≥ 50%, stärker bevorzugt ≥ 60%, noch stärker bevorzugt ≥ 70% der Gesamtoberfläche der Membran aus.

Die Geometrie der Strukturen auf der Membran kann beliebig variiert werden. So können Kanäle und/oder Verästelungen als Strukturen auf der Membran verwendet werden, die beispielsweise die Kapillarstruktur der natürlichen Lunge nachahmen.

Solche Kanäle und/oder Verästelungen können auch dadurch erhalten werden, dass die Membran durch verschiedene geometrische Formen strukturiert wird. Beispiele für vorteilhafte geometrische Formen sind Rauten, Vierecke, Vielecke und Kreise. Die Kanäle können beispielsweise gerade sein aber sie können ebenfalls nicht linear sein, wie z.B. in einer geschlängelten Form oder in einer Mischerstruktur.

Wie schon beschrieben, wird die Membran aufgrund der Strukturierung mit herausragenden, erhabenden Bereichen bereitgestellt. Diese Bereiche weisen eine bevorzugte Höhe von ca. 1 bis 100 µm, bevorzugt von ca. 5 bis 50 µm, stärker bevorzugt von ca. 10 bis 30 µm und am stärksten bevorzugt von ca. 10 bis 20 µm, auf.

Die entstehenden Kanäle und/oder Verästelungen auf der Membran können entweder ganz durchgängig sein und einen konstanten Fluss des Mediums ermöglichen, sie können aber auch nur abgeschlossene Verzweigungen darstellen, in die das Medium eindringt und (ggf. nach dem Gastransfer) auf dem gleichen Weg wieder austritt. Bevorzugt ist die Membran überwiegend mit Kanälen ausgestattet, noch stärker bevorzugt überwiegend mit Kanälen und Verästelungen.

Erfindungsgemäß stellen die Kanäle auf der Membran durchgängige Wege für ein Medium dar, so dass das Medium parallel, antiparallel oder in einer anderen Form (wie bspw. wellenförmig) zur Einströmrichtung in die Kammer durch die Kanäle hindurchgleiten kann. Hingegen sind Verästelungen erfindungsgemäß Verzweigungen, in die ein Medium eintreten kann, jedoch wie in einer Sackgasse nicht die Verzweigung an einer anderen Stelle als der Eintrittsstelle wieder verlassen kann. Die Verästelungen können parallel oder senkrecht zur Einströmrichtung in die Kammer angeordnet sein. Sie können auch jeden beliebigen Winkel zwischen einer parallelen oder senkrechten Anordnung einnehmen, d.h. einen Winkel von 0° bis 90°. Vorzugsweise sind die Verästelungen in einem Winkel von ca. 10° bis 80°, stärker bevorzugt von ca. 20° bis 70°, noch stärker bevorzugt von ca. 30° bis 60° und am stärksten bevorzugt von ca. 40° bis 50°, zur Einströmrichtung angeordnet. Bei einer solchen Anordnung der Verästelungen gegenüber der Einströmrichtung in die Kammer liegen die Eingänge der Verästelungen auf der dem Hauptstrom zugewandten Seite. Der Einstrom in die Verästelungen findet dabei parallel, senkrecht oder in einem der oben genannten Winkel zum Hauptstrom statt. Es ist ebenfalls eine Anordnung der Verästelungen zur Einströmrichtung denkbar, die eine Anordnung zwischen einer senkrechten und parallelen liegt, so dass der Einstrom in die Verästelungen im Wesentlichen antiparallel (d.h. antiparallel oder in einem beliebigen Winkel zwischen dem antiparallelen und senkrechten Einstrom) zum Hauptstrom stattfindet.

Es hat sich als besonders vorteilhaft herausgestellt, wenn die für die Kanäle und/oder Verästelungen zur Verfügung stehende Oberfläche mehr als 50% der Gesamtoberfläche der Membran ausmacht. Dieser Anteil kann leicht mathematisch berechnet werden, indem der Differenzwert (in m²) aus der Gesamtoberfläche der Membran und der Oberfläche, die mit Strukturen belegt ist, mit dem Wert (in m²) für die Gesamtoberfläche ins Verhältnis gesetzt wird.

Die in der erfindungsgemäßen Gastransfervorrichtung verwendete Membran kann aus beliebigen Materialien bestehen, die eine gute Gaspermeabilität (Gasdurchlässigkeit) aufweisen. Gute Gaspermeabilitäten ist beispielsweise gegeben bei Werten von über 100 ml/m², bevorzugt über 1.000, stärker bevorzugt über 5.000, noch stärker bevorzugt über 10.000 und am stärksten bevorzugt über 20.000 ml/m² pro Stunde bei 24 atm (d.h. über 20.000 ml/m² innerhalb von 24h bei Umgebungsdruck), und bei 25°C, 90% relativer Feuchte (RH) und einer Materialstärke von ca. 30 µm, je nach gewünschtem Zweck in Hinblick auf das jeweils gewünschte Gas (insbesondere bspw. Sauerstoff oder Kohlendioxid). Ferner ist die Membran typischerweise im Wesentlichen flüssigkeitsundurchlässig, d.h. sie besitzt eine Feuchtigkeitspermeabilität von < 1.000, bevorzugt < 500, stärker bevorzugt < 100 und noch stärker bevorzugt < 10 g/m² (i.e. gH₂O/m²) in 24 h, 40°C, 90% RH.

Die (gas-)permeable Membran kann einerseits eine poröse Membran sein, d.h. eine Membran, die diskrete Poren aufweist. Andererseits kann die Membran eine homogene Löslichkeitsmembran ohne diskrete Poren sein, in der der Stofftransport durch Lösung des Permeats (d.h. Gases) im Polymer erfolgt und die Trennung aufgrund unterschiedlicher Löslichkeiten im Polymer stattfindet. Vorzugsweise ist die Membran eine nicht-poröse permeable Membran. Der Gasaustausch kann dem konvektiven und diffusiven Stoffaustausch unterliegen. Vorzugsweise ist der Gasaustausch diffusiv und wird über die Differenz der Gaskonzentration auf beiden Seiten der Membran bestimmt.

In einer spezifischen Ausführungsform der vorliegenden Erfindung ist die Membran selektiv im Wesentlichen für Sauerstoff und/oder Kohlenstoffdioxid durchlässig. Abhängig von dem Einsatzort der erfindungsgemäßen Gastransfervorrichtung kann die Membran für bestimmte Gase besonders gut durchlässig sein, die Permeabilität kann für andere Gase hingegen eingeschränkt sein. Beispielsweise bei der Verwendung der Gastransfervorrichtung zum Gasaustausch im Blut ist eine gute Permeabilität für Sauerstoff und/oder Kohlenstoffdioxid von Bedeutung (siehe die oben genannten Permeabilitätswerte).

In einer anderen spezifischen Ausführungsform ist die Membran nicht oder nur gering für Stickstoff durchlässig. Bspw. für eine Anwendung zum Gasaustausch ist es von Vorteil, wenn die Membran nur geringfügig für Stickstoff durchlässig ist. Dann kann das Blut mit Luft (anstelle von reinem Sauerstoff) begast werden.

Für andere Anwendungen der erfindungsgemäßen Gastransfervorrichtung wird die Membran gute Permeabilitäten für andere Gase aufweisen. Beispielsweise im Einsatz als bzw. für Reaktoren (z.B. Bioreaktoren) kann die Membran eine gute Permeabilität für ein oder mehrere Gase, ausgewählt aus N₂, O₂, CO₂, H₂, NH₃, H₂S, CH₃ oder andere Kohlenwasserstoffe, oder auch andere Gase (bspw. Edelgase) aufweisen. Die Membranpermeabilität für bestimmte Gase beim Einsatz der erfindungsgemäßen Gastransfervorrichtung in der Chemie, z.B. zur Gasaufreinigung, Gastrennung oder für Reaktionen, hängt dabei von der Art des zu trennenden bzw. aufzureinigenden Gases bzw. von der Art des der Reaktion zugeführten bzw. abgeführten Gases ab.

Es liegt dabei im Ermessen des Fachmanns, ein Membranmaterial mit der geeigneten spezifischen Permeabilität für ein bestimmtes Gas oder bestimmte Gase bei der angestrebten Verwendung der erfindungsgemäßen Gastransfervorrichtung auszuwählen. Permeabilitätswerte für viele Membranmaterialien sind aus dem Stand der Technik bekannt.

Die Membranstärke beträgt erfindungsgemäß ca. 1-200 µm, bevorzugt im Bereich von ca. 5-200 µm, weiter bevorzugt ca. 10-100 µm, stärker bevorzugt ca. 20-50 µm. Dabei handelt es sich um die Stärke der Membran ohne die durch die Strukturierung erhaltenen herausragenden Bereiche.

Durch die geringe Stärke der Membran kann es notwendig sein, die Membran durch ein geeignetes Trägermaterial zu stabilisieren. Vorzugsweise wird die Membran durch ein Trägermaterial, ausgewählt aus der Gruppe, bestehend aus porösen Schäumen, Keramiken, Polymeren ggf. auch einer Stützschicht aus TPX stabilisiert.

Gemäß einer weiteren Ausführungsform der vorliegenden Erfindung kann die Membran auch als Membranstapel ausgestaltet sein. Die Verwendung von Membranstapeln hat typischerweise mindestens zwei Vorteile gegenüber der Verwendung einfacher Membranen. Einerseits wird der Wirkungsgrad erhöht, da mehr Oberfläche für den Gasaustausch zur Verfügung steht. Andererseits sind Membranstapel auch stabiler als einfache Membranen. Hingegen ist bei Anwendungen, die eine kompakte Gestaltung der erfindungsgemäßen Gastransfervorrichtung erforderlich machen, bspw. infolge einer Miniaturisierung, die Wahl von Membran anstelle von Membranstapeln stärker bevorzugt.

Das Stapeln von Membranen kann automatisiert oder von Hand durchgeführt werden. Die Membranen können dabei entweder parallel übereinander gelegt werden oder um einen bestimmten Winkel gegeneinander versetzt sein. Besonders bevorzugt sind Membranstapel, die einen gegeneinander versetzten Winkel von 90° aufweisen. Die einzelnen Membranen werden am Randstreifen und/oder an jeder Erhöhung miteinander verbunden. Dabei können Techniken wie Kleben (z.B. UV-Kleber), Ultraschallverschweißen, Hitze-Verschweißen, Bonden oder die Ausbildung von kovalenten molekularen Verbindungen (beispielsweise NH₃-COOH zur Amidbindung) zum Einsatz kommen.

Die Membranstapel können entweder gleiche oder unterschiedliche Membranen im Hinblick auf deren Strukturierung oder Zusammensetzung aufweisen. In einer bevorzugten Ausführungsform bestehen die Membranstapel aus strukturierten, permeablen, nicht-porösen Membranen in alternierender Abfolge mit nanoporösen Membranen. Vorzugsweise bestehen die Membranstapel aus ca. 10, bevorzugt ca. 50, stärker bevorzugt ca. 100 und noch stärker bevorzugt mehr als ca. 100 Membranen, z.B. sogar über 1000 Membranen.

Die kritische Komponente der erfindungsgemäßen Gastransfervorrichtung im Hinblick auf ihre Haltbarkeit ist die Membran. Der bisherige vielseitige klinische Einsatz von organunterstützenden Systemen mit Fremdoberflächen, die mit Blut in Berührung kommen, hat gezeigt, dass es zu unerwünschten systemischen Reaktionen (proinflammatorische Immunantwort) kommen kann. Bei der Langzeitanwendung herkömmlicher Blutkontaktflächen führt eine Anlagerung von Plasmaproteinen und Zellen zur Querschnittsverengung und Thrombosebildung. Zudem wird bei langfristigem Einsatz die Bildung einer proliferativen Innenschicht bewirkt, was als "Neo-Intima" bezeichnet wird. Insbesondere wird dieses Phänomen bei Oxygenatoren, die zur Unterstützung der Lungenfunktion in beispielsweise Herz-Lungen-Maschinen Verwendung finden, aber auch bei Kunstherzsystemen bzw. Herzunterstützungssystemen oder Hämodialyseapparaturen beobachtet. Deshalb kann und sollte die Membran auf unterschiedliche Weise in ihrer Haltbarkeit verbessert werden.

In einer Ausführungsform der vorliegenden Erfindung kann die Membran durch Plasmaaktivierung nachbehandelt werden.

In einer weiteren Ausführungsform der vorliegenden Erfindung, insbesondere für die Verwendung der erfindungsgemäßen Gastransfervorrichtung in der Medizin, kann die Membran mit Zellen, vorzugsweise Ephitelzellen, besiedelt sein. Für eine langfristig einsetzbare erfindungsgemäße Gastransfervorrichtung im medizinischen Rahmen (beispielsweise als Lungenunterstützungssystem) stellt die Besiedlung der Membran mit Zellen eine deutliche Verlängerung der Haltbarkeit dar, da dadurch eine unspezifische Anlagerung von Substanzen aus den verwendeten Medien an die Membran vermieden bzw. stark gehemmt wird und somit die Membran in ihrer Gaspermeabilität mit der Zeit nicht oder nur unwesentlich verschlechtert wird.

In einer noch weiteren Ausführungsform der vorliegenden Erfindung ist die Membran mit biologischen Substanzen, bspw. ausgewählt aus (Poly)Saccharid, vorzugsweise Heparin, Nukleinsäure, vorzugsweise DNA, RNA oder PNA, Protein, vorzugsweise Albumin, Lipid, Proteoglykan, oder organischen Polymeren, bspw. Polyethylenglycol oder mit Kombinationen dieser Substanzen beschichtet.

Zur Besiedlung der Membran mit Zellen bzw. zur Beschichtung der Membran mit anderen Substanzen kann es vorteilhaft oder auch notwendig sein, die Oberfläche der Membran vorher zu modifizieren. Verfahren zur Modifikation von Membranen sind aus dem Stand der Technik bekannt und können vom Fachmann anwendungsbezogen ausgewählt werden. Beispielsweise kann es notwendig sein, die Hydrophobizität / Hydrophilie / Ladungsdichte der Membran zu verändern, beispielsweise durch physikalische oder chemische Behandlung der Membran, um die Anhaftung zu verbessern.

Die Erfindung betrifft eine Gastransfervorrichtung umfassend eine wie oben beschrieben strukturierte Membran.

Erfindungsgemäß umfasst der Begriff "Gastransfervorrichtung" eine Begasungs- bzw. Entgasungs- und Gasaustauschvorrichtung. Bei einer Begasungs- bzw. Entgasungsvorrichtung treten ein oder mehrere Gase aus einer Kammer in die andere Kammer über, ohne dass ein Rücktransport stattfindet. Bei einer Gasaustauschvorrichtung hingegen wandern zusätzlich aus der anderen Kammer das gleiche oder ein anderes Gas oder auch mehrere Gase in die erste Kammer ein. Die Gase werden also gegeneinander ausgetauscht. Dabei muss die Gasmenge, die in die eine Richtung wandert, mit der Gasmenge, welche eine entgegengerichtete Wanderung vollzieht, nicht identisch sein. Typischerweise erfolgt der Transfer nach Maßgabe des jeweiligen Konzentrationsgefälles.

Die mindestens zwei Kammern der erfindungsgemäßen Gastransfervorrichtung dienen zur Aufnahme jeweils eines Mediums, d.h. eines Gases, einer Flüssigkeit oder eines Feststoffs bzw. deren Gemischen. In Abhängigkeit vom Einsatzgebiet für die erfindungsgemäße Gastransfervorrichtung dienen die Kammern zur Aufnahme spezieller Medien. Bei einer Anwendung zur Gasaufreinigung ist ein abzutrennendes Gas in einem Gasgemisch oder in einer Flüssigkeit in einer der Kammern enthalten, eine weitere Kammer enthält ebenfalls entweder ein Gas oder eine Flüssigkeit, in die das zu trennende Gas übergehen soll. Die zweitgenannte Kammer kann aber auch ungefüllt sein bzw. unter einem leichten oder starken Unterdruck stehen, um den Gastransfer zu erleichtern.

Zur Verwendung in der Biotechnologie dient mindestens eine der Kammern vorzugsweise zur Aufnahme einer Flüssigkeit, z.B. eines Kulturmediums, und eine andere Kammer zur Aufnahme eines Gases, das in die Flüssigkeit übergehen soll. In seiner Verwendung als Oxygenator in der Medizin umfasst die erfindungsgemäße Gastransfervorrichtung mindestens zwei Kammern, wobei vorzugsweise eine der Kammern zur Aufnahme einer Flüssigkeit und eine weitere Kammer zur Aufnahme eines Gases dient, das in die Flüssigkeit übergehen soll. Wird die erfindungsgemäße Gastransfervorrichtung zur Begasung oder zum Gasaustausch im Blut verwendet, so nimmt mindestens eine Kammer die Flüssigkeit, also bspw. Blut, auf und mindestens eine weitere Kammer nimmt Sauerstoff oder ein Sauerstoff-enthaltendes Gasgemisch auf. Dabei geht durch die Membran Sauerstoff aus der einen Kammer in die mit Blut gefüllte Kammer über, und optional tritt Kohlenstoffdioxid aus der mit Blut gefüllten Kammer in die Sauerstoff-enthaltende Kammer ein. Hierbei kann der Transfer von Kohlenstoffdioxid in die mindestens eine Sauerstoff-enthaltende Kammer auch unterbleiben, indem die Membran so gewählt wird, das sie keine Kohlenstoffdioxid-Permeabilität zulässt.

Abhängig von der Verwendung der erfindungsgemäßen Gastransfervorrichtung können die Kammern aus jedem beliebigen geeigneten Material hergestellt werden. Beispielsweise können die Kammern aus Kunststoff, Metall, Glas, Keramik oder anderen Materialien, bspw. Komposit-Werkstoffen, bestehen. Auch können zur Verwendung in Reaktoren Stahlkammern eingesetzt werden. Bevorzugtes Material für die Kammern ist Kunststoff. Es liegt jedoch im Ermessen des Fachmanns, für eine bestimmte Verwendung der erfindungsgemäßen Gastransfervorrichtung ein geeignetes Material für die Kammern auszuwählen. Dabei können die unterschiedlichen Kammern entweder aus dem gleichen Material oder auch aus unterschiedlichen Materialien gefertigt sein. Für eine chemische Anwendung ist dabei insbesondere die Stabilität des Materials von Bedeutung, hingegen muss für die Verwendung in der Biotechnologie und Medizin insbesondere auf die Verträglichkeit mit den in den Kammern verwendeten Medien geachtet werden, insbesondere müssen die Standards für die bspw. medizinischen Anwendung (Sterilität etc.) eingehalten werden.

Ebenfalls abhängig von der Verwendung der erfindungsgemäßen Gastransfervorrichtung kann die Größe der Kammern in geeigneter Weise gewählt sein. Dabei können die Kammern gleich oder unterschiedlich groß sein und auch gleiche oder unterschiedliche Geometrien aufweisen. So können eine oder auch mehrere Kammern so klein dimensioniert sein, dass die Membran direkt mit der (gegenüberliegenden) Kammerwand in Verbindung steht (bevorzugt über die Strukturmuster auf der Membran). Andererseits können auch eine der Kammern oder auch mehrere Kammern Dimensionen von bis zu einigen Metern aufweisen. Beispielsweise bei der Verwendung als Reaktor (z.B. in der Chemie oder Biotechnologie) kann eine der Kammern als Reaktor selbst ausgestaltet sein, der beispielsweise bis zu etwa 10 m breit ist. Im erstgenannten Fall einer kleinen Kammerdimensionierung weist mindestens eine der Kammern vorzugsweise einen Durchmesser von etwa 1 µm bis etwa 1 cm, stärker bevorzugt etwa 5 µm bis etwa 500 µm, noch stärker bevorzugt etwa 10 µm bis etwa 200 µm und am stärksten bevorzugt etwa 20 µm bis etwa 100 µm auf. Im zweitgenannten Fall, bspw. bei der Verwendung als Reaktor, weist mindestens eine der Kammern vorzugsweise einen Durchmesser von etwa 1 cm bis etwa 10 m, stärker bevorzugt etwa 5 cm bis etwa 5 m, noch stärker bevorzugt etwa 10 cm bis etwa 2 m und am stärksten bevorzugt etwa 20 cm bis etwa 1 m auf. Besonders bevorzugt besitzen mindestens zwei Kammern die oben genannten Durchmesser. Es liegt jedoch im Ermessen des Fachmanns, für eine spezifische Verwendung der erfindungsgemäßen Gastransfervorrichtung eine geeignete Größe für die Kammern zu bestimmen. Dies gilt ebenfalls für die Dimensionierung der Länge und der Höhe der Kammern.

Die Kammern der erfindungsgemäßen Gastransfervorrichtung weisen jeweils mindestens eine Öffnung zur Aufnahme des Mediums auf. Vorzugsweise weisen die Kammern jeweils mindestens einen Ein- und Auslass auf, um als Durchflusskammern ausgestaltet zu sein. Vorzugsweise ist mindestens eine der Kammern als Durchflusskammer ausgestaltet, stärker bevorzugt sind alle Kammern als Durchflusskammern ausgestaltet. An den Ein- und/oder Auslässen können beispielsweise Anschlüsse zu weiteren Kammern oder Geräten vorgesehen sein. Beispielsweise können an den Ein- und/oder Auslässen Anschlüsse für Schläuche, welche zum Einleiten der Medien in die Kammern und/oder zur Abführung der Medien dienen, vorgesehen sein. Wenn mindestens zwei Kammern als Durchflusskammern ausgestaltet sind, können sie im Gleich- oder Gegenstrom betrieben werden. Zum Durchleiten der Medien durch die Kammern können beispielsweise Pumpen verwendet werden. Das Durchleiten der Medien durch die Kammern kann unter Umgebungsdruck oder bei Unter- oder Überdruck geschehen. Zum Beispiel könnte das Durchleiten der Medien durch die Kammern bei einem Unterdruck von 100 bis 10 mbar oder bei einem Überdruck von 50 bis 300 mbar geschehen.

In einer Ausführungsform umfasst die Gastransfervorrichtung mehr als zwei, vorzugsweise mehr als 10, stärker bevorzugt mehr als 20, noch stärker bevorzugter mehr 50, am stärksten bevorzugt mehr als 100 Kammern, die jeweils durch eine Membran voneinander getrennt sind.

Vorzugsweise ist die erfindungsgemäße Gastransfervorrichtung so aufgebaut, dass die Kammern abwechselnd das Medium enthalten, welches ein Gas aufnimmt und welches ein Gas abgibt bzw. ein Gas ist.

Eine erfindungsgemäße Gastransfervorrichtung kann weiterhin, in einer alternativen Ausführungsform der Erfindung, aus zwei oder mehreren der genannten Vorrichtungen bestehen, wobei die Kammern vorzugsweise übereinander (d.h. parallel zueinander) gelagert sind. Alternativ können die Kammern auch konzentrisch seriell oder umeinander angebracht sein.

Vorzugsweise wechseln sich auch hier die Kammern mit dem Medium, welches das Gas aufnimmt, mit Kammern, welche das Gas abgebende Medium beinhalten, ab.

Eine Vorrichtung umfassend mehr als zwei Kammern gewährleistet einen größeren Wirkungsgrad als Vorrichtungen mit nur zwei Kammern. Dem verbesserten Wirkungsgrad steht ein Verlust der Kompaktheit der erfindungsgemäßen Gastransfervorrichtung gegenüber.

Erfindungsgemäß findet die Gastransfervorrichtung Anwendung für die Begasung bzw. den Gasaustausch in beliebigen Medien (Gas, Flüssigkeit, Feststoff, Gemischen davon, etc.).

In der Chemie kann die erfindungsgemäße Gastransfervorrichtung im Allgemeinen für Reaktionen verwendet, an denen Gase beteiligt sind, wie z.B. Gas/Gas-, Gas/Flüssig- oder Gas/Feststoff-Reaktionen. Ferner kann die erfindungsgemäße Gastransfervorrichtung ebenfalls zur Gasaufreinigung und Gastrennung verwendet werden.

Bevorzugt wird die Gastransfervorrichtung in der Biotechnologie und in der Medizin eingesetzt. In der Biotechnologie kommt sie insbesondere zum Einsatz als oder im Bioreaktor zur Kultivierung diverser Zellen bspw. mit dem Ziel einer Expression von Genen von Interesse.

In der Medizin wird die erfindungsgemäße Gastransfervorrichtung bevorzugt zur Begasung von Blut eingesetzt, insbesondere bei Patienten mit Lungenversagen oder anderen Lungenfehlern zur Dauertherapie oder bei Operationen, z.B. Transplantationen, bei denen der Patient an eine Herz-Lungen-Maschine angeschlossen wird, zur Akuttherapie.

Bei einer besonders bevorzugten Verwendung kann die erfindungsgemäße Gastransfervorrichtung zur Begasung von Blut bzw. zum Gasaustausch in Blut eingesetzt werden. Sie übernimmt dadurch Funktionen einer künstlichen Lunge. Eine solche künstliche Lunge wird typischerweise als externe Vorrichtung ausgestaltet sein, sie könnte aber auch in einen Patienten implantiert sein. Abhängig davon, ob die erfindungsgemäße Gastransfervorrichtung als externe oder implantierte Vorrichtung ausgestaltet ist, besitzt sie unterschiedliche Dimensionen. So wird eine intern einsetzbare erfindungsgemäße Gastransfervorrichtung im Miniaturmaßstab strukturiert sein, damit sie insbesondere zur Implantation, z.B. in die Vene eines Patienten, geeignet ist.

Abhängig von der Verwendung der erfindungsgemäßen Gastransfervorrichtung können die Kammern der Gastransfervorrichtung zur Aufnahme unterschiedlicher Medien dienen. Beispielsweise können mehrere Kammern mit Gasen befüllt sein. Eine der Kammern oder auch alle Kammern können ebenfalls zur Aufnahme einer Flüssigkeit dienen.

Bei der Verwendung in der Medizin oder in der Biotechnologie ist die Flüssigkeit bevorzugt eine biologische Flüssigkeit. Die biologische Flüssigkeit soll sowohl als Flüssigkeit verstanden werden, welche eine Körperflüssigkeit eines Lebewesens ist, als auch als eine Flüssigkeit, welche biologisch für mindestens einen Organismus ungiftig ist bzw. für dessen Wachstum benötigt wird. In einer Ausführungsform der vorliegenden Erfindung ist die biologische Flüssigkeit ausgewählt aus der Gruppe, bestehend aus Blut, Blutserum, Zellsuspension, Zelllösung und Kulturmedium. Dient mindestens eine der Kammern der erfindungsgemäßen Gastransfervorrichtung zur Aufnahme einer Flüssigkeit, so ist es bevorzugt, dass die Membran, welche diese Kammer von einer weiteren Kammer trennt, auf der Seite der Flüssigkeit eine Strukturierung, wie oben beschrieben, aufweist.

Wie bereits beschrieben, können die Kammern der erfindungsgemäßen Gastransfervorrichtung unterschiedlich dimensioniert sein. So können die Kammern sehr geringe Dimensionen aufweisen und als Durchflusskammern zum Einsatz beispielsweise in Reaktoren (Chemie- oder Bioreaktoren) eingesetzt werden. Dabei können sie entweder intern oder auch extern, über geeignete Verbindungen, an die Reaktoren angeschlossen sein. Beispielsweise kann ein Teil eines Mediums über geeignete Verbindungen (z.B. Schläuche) aus dem Reaktor abgeführt werden und in eine der Kammern der Gastransfervorrichtung eingeleitet werden. Die andere Kammer (beispielsweise als Durchflusskammer ausgestaltet) kann mit einem anderen Medium befüllt werden, welches ein Gas an die Flüssigkeit aus dem Reaktor abgibt oder ein solches aufnimmt.

Andererseits kann mindestens eine der Kammern so große Dimensionen aufweisen, dass sie selbst als Reaktor dient. Durch eine weitere Kammer wird dann ein Medium geleitet, welches zur Aufnahme bzw. zur Abgabe eines bestimmten Gases an das Reaktormedium dient.

Eine beispielhafte Realisierung einer extrakorporalen Gastransfervorrichtung zur Begasung von Blut könnte in Anlehnung an den Novalung^{®}-iLA Membran Ventilator IL-1000-01 der Novalung GmbH realisiert werden. Dieser Ventilator gehört zur Gruppe der extrakorporalen Gasaustauschsysteme, die eine Albumin-Heparin-Beschichtung beinhalten. Ein solcher Ventilator dient zur Zufuhr von Sauerstoff und zu Abfuhr von Kohlenstoffdioxid von abgeleitetem Blut eines Patienten. Das System besteht aus einer sog. künstlichen Lunge, welche einer Gastransfervorrichtung entspricht, und blutzu- bzw. blutabführenden Ein-/Auslassleitung mit Schlauchverlängerung. Dieses System ist richtungsunabhängig und kann aufgrund seines symmetrischen Aufbaus von beiden Seiten angeströmt werden. Die beiden Ein-/Auslassleitungen und Schlauchverlängerungen des Systems sind aus folgenden Komponenten aufgebaut:
- Ein-/Auslassbogen als Knickschutz
- PVC-Schlauch 3/8x3/32 Zoll mit Schnellkupplung weiblich
- Schlauchverlängerung mit Schnellkupplung männlich/weiblich

Die blutzu-/blutabführenden Ein-/Auslassbögen sind als Knickschutz im Ein-/ Auslassbereich der Membranlunge angebracht. Die Übergangsstellen in den Ein-/Auslassleitungen und Schlauchverlängerungen sind stufenlos gestaltet, um die Gefahr einer Thrombenbildung durch Totzonen, scharfe Kanten etc. im Strömungsbereich zu minimieren.

Die bei dem Novalung^{®}-iLA Membran Ventilator nach dem Stand der Technik verwendete Membran ist eine Hohlfasermembran, über die Sauerstoff an das Blut abgegeben wird und Kohlenstoff aus dem Blut abgeführt wird. Erfindungsgemäß kann diese Hohlfasermembran durch eine strukturierte Membran im Sinne der vorliegenden Erfindung ausgetauscht werden.

Das Novalung^{®}-iLA Membran Ventilator-System funktioniert nach dem folgenden Prinzip: Das Blut tritt aus der Arteria femoralis über die arterielle Novalung^{®}-Kanüle NovaPort^{®} in die zuführende Schlauchverlängerung und Einlassleitung ein. Das Blut tritt durch den Einlaufbogen in das Gehäuse der Membranlunge ein. In der dahinterliegenden Vorkammer wird das Blut verteilt, gleichzeitig wird hier eventuell eintretende Luft nach oben abgeleitet. An der Spitze des Membransystems sind beidseitig Entlüftungsmembranen integriert. Diese sind hydrophobe Membranen, die gasförmige Stoffe durchlassen, Flüssigkeiten jedoch zurückhalten. Die Entlüftungsmembranen dienen dem leichteren Füllen, Entlüften und der permanenten Elimination von Luft während des Verfahrens. In der folgenden Hauptkammer findet wie oben beschrieben der Gasaustausch statt.

Das decarboxylierte und oxygenierte Blut wird dem Patienten über den Auslaufbogen, das Membransystem, die Auslassleitung mit Schlauchverlängerung und die Novalung^{®}-Kanüle NovaPort^{®}der Vena Femoralis zugeführt.

Weitere technische Daten sind in der folgenden Tabelle angegeben:

| Technische Daten: | | |
|---|---|---|
| Blutflussrate | 0,5 - 4,5 l/min | |
| Maximal empfohlene Gasflussrate | 10 l/min | |
| Maximaler Druck Gasseite | 20 mmHg | |
| Maximaler Druck Blutseite | 200 mmHg | |
| Oberfläche der Oxygenationsmembran | 1,3 m² | |
| Füllvolumen gesamt | 240 ml | |

| Periphere Schnittstellen: | | |
|---|---|---|
| Anschlussstelle | Anschlußports | Anschlußgröße |
| Blutein-/Blutauslassschlauch | 2 | 3/8"x1/32" Schnellkupplungen |
| Gaskonnektoren | 2 | 1/4" |
| Entlüftungskonnektoren | 2 | Luer-Lock |

Die erfindungsgemäße Gastransfervorrichtung kann ferner noch weitere Bauteile umfassen. Dazu gehört beispielsweise ein Gehäuse, welches aus einem beliebigen Material aufgebaut sein kann.

Ferner können an die erfindungsgemäße Gastransfervorrichtung noch weitere Bauteile angeschlossen sein, die für eine gute Funktionsweise der Gastransfervorrichtung notwendig sind bzw. diese positiv unterstützen. Beispielsweise kann ein Wärmetauscher an die Gastransfervorrichtung angeschlossen sein, um die Temperatur des in den Kammern befindlichen bzw. durch die Kammer durchgeleiteten Mediums zu temperieren. Weiterhin können an die erfindungsgemäße Gastransfervorrichtung ebenfalls Apparaturen angeschlossen werden, welche bestimmte Parameter des in den Kammern befindlichen bzw. durch die Kammer durchgeleiteten Mediums überwachen bzw. vorgeben. Beispielsweise kann eine Apparatur an die Gastransfervorrichtung angeschlossen sein, welche den Gasdruck (bei Verwendung eines Gases als Medium) überwacht.

### Kurze Beschreibung der Zeichnungen

Die Erfindung wird nun anhand bestimmter in den beigefügten Zeichnungen dargestellter Ausführungsbeispiele erläutert, in denen gleiche Merkmale mit den gleichen Bezugszeichen versehen sind. Es zeigt:
- Figur 1: eine schematische perspektivische Ansicht einer strukturierten Membran zur Anwendung in einer Gastransfervorrichtung gemäß einem einfachen Ausführungsbeispiel der Erfindung,

- Figur 2: einen schematischen Querschnitt durch einen Teil einer Gastransfervorrichtung gemäß einem einfachen Ausführungsform der Erfindung,
- Figur 3: eine perspektivische Ansicht einer strukturierten Membran zur Anwendung in einer Gastransfervorrichtung gemäß einem bevorzugten Ausführungsbeispiel der Erfindung,
- Figur 4: eine perspektivische Ansicht eines Teils der in Figur 3 gezeigten, strukturierten Membran,
- Figur 5: eine weitere perspektivische Ansicht eines Teils der in Figur 3 gezeigten, strukturierten Membran,
- Figur 6: eine perspektivische Ansicht einer Gastransfervorrichtung gemäß einem bevorzugten Ausführungsbeispiel der Erfindung, und
- Figur 7: eine Graphik der Anreicherung von Sauerstoff in einer Testflüssigkeit in einer einfachen Gastransfervorrichtung gemäß einem Ausführungsbeispiel der Erfindung.

### Beschreibung der Ausführungsbeispiele der Erfindung

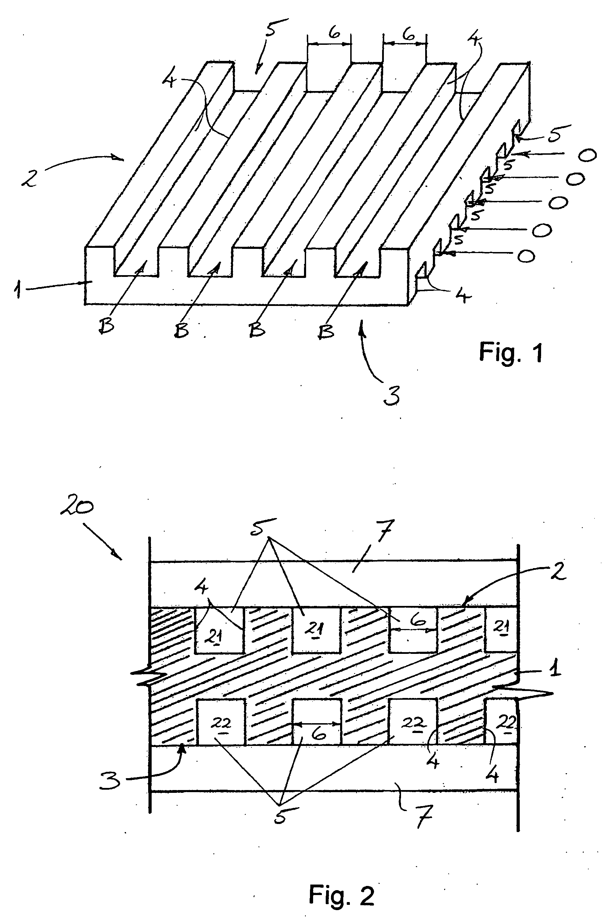

In Figur 1 ist ein Teil einer gasdurchlässigen und flüssigkeitsundurchlässigen, strukturierten Membran 1 zur Anwendung in einer Gastransfervorrichtung gemäß einem einfachen Ausführungsbeispiel der Erfindung schematische und stark vergrößert dargestellt. Die beiden gegenüber liegenden Seiten 2, 3 der Membran 1 sind mit einer Struktur versehen und diese Struktur besteht aus parallel verlaufenden Wänden 4, die dazwischen Kanäle 5 bilden. Die Wände 4 sind in regelmäßigen Abständen 6 voneinander über die beiden äußeren Flächen 2, 3 der Membran 1 angeordnet und ragen im Wesentlichen senkrecht nach oben bzw. nach unten aus der Ebene der Membran 1 heraus. Die Beabstandung 6 der Wände 4 in diesem Ausführungsbeispiel liegt im Bereich von ca. 20 bis 150 µm und die Kanäle 5 machen über 50 % der Gesamtoberfläche 2, 3 der Membran 1 aus. Wie der Figur 1 auch zu entnehmen ist, erstrecken sich die Kanäle 5 auf der oberen Seite 2 der Membran 1 quer bzw. rechtwinkelig zu den Kanälen 5 auf der unteren Seite 3 der Membran.

In einer einfachen erfindungsgemäßen Gastransfervorrichtung werden mindestens zwei Kammern durch die in Figur 1 gezeigte, gasdurchlässige und flüssigkeitsundurchlässige Membran voneinander getrennt. Auf der oberen Seite 2 der Membran 1 wird mindestens eine erste Kammer teilweise von der Membran 1 gebildet und auf der unteren Seite 3 wird mindestens eine zweite Kammer teilweise von der Membran 1 gebildet. Die mindestens eine erste Kammer auf der oberen Membranseite 2 ist zur Aufnahme einer biologischen Flüssigkeit wie z.B. Blut bestimmt und die mindestens eine zweite Kammer auf der unteren Membranseite 3 ist zur Aufnahme eines Gases wie z.B. Sauerstoff bestimmt. Jede Kammer ist ferner als eine Durchflusskammer ausgebildet, sodass das Blut B oben z.B. von vorne nach hinten und der Sauerstoff O unten z.B. von rechts nach links durch die jeweilige Kammer fließen kann. Wie oben beschrieben, dient die Strukturierung der Membranoberflächen dem verbesserten Gastransfer, in dem eine größere Fläche für den intimen Kontakt zwischen den flüssigen und gasförmigen Phasen bereitgestellt wird. Zudem können aber auch die Flusseigenschaften der flüssigen und gasförmigen Phasen über die Strukturierung (z.B. durch Turbulenzen) den Gasübertritt über die Membran verstärken. Ferner entsteht ein verbesserter Stofftransport durch insbesondere verbesserte Diffusionseigenschaften, die auf die kleine Beabstandung 6 der Wände 4 und damit die Herabsetzung des Flusswiderstandes zurückgeht.

In Figur 2 wird ein schematischer Querschnitt durch einen Teil einer Gastransfervorrichtung 20 gemäß einem einfachen Ausführungsbeispiel der Erfindung gezeigt. Die Membran 1 in diesem Beispiel wird durch zwei Schichten 7 von einem Trägermaterial stabilisiert. Das Trägermaterial 7 besteht vorzugsweise aus dem gleichen Polymer als die Membran 1 und wird mit ihr durch ein Verschweißen (z.B. mit Ultraschall oder Laser) oder eine kovalente Bindung verbunden. Jede Trägerschicht 7 kann die gleiche oder eine größere Wandstärke haben als die Membran 1, z.B. eine deutlich größere Wandstärke bis zu 1 mm oder 2 mm. Wie in Figur 2 klar zu sehen, bilden die Kanäle 5 auf der oberen Seite 2 der Membran 1 zusammen mit der oberen Trägerschicht 7 eine Reihe von nebeneinander angeordneten, ersten Kammern 21 für die biologische Flüssigkeit (Blut). In gleicher Weise bilden die Kanäle 5 auf der unteren Seite 3 der Membran 1 zusammen mit der unteren Trägerschicht 7 eine Reihe von nebeneinander angeordneten, zweiten Kammern 22 für das Gas (Sauerstoff). In diesem Beispiel verlaufen die Kanäle 5 auf den oberen und unteren Seiten 2, 3 der Membran 1 parallel miteinander aber dafür fließen das Blut B und der Sauerstoff O in den entgegen gesetzten Richtungen. Auf der oberen Seite 2 z.B. fließt das Blut B durch die ersten Kammern 21 vom Betrachter gesehen in die Zeichnungsebene hinein und auf der unteren Seite fließt der Sauerstoff O durch die zweiten Kammern 22 aus der Zeichnungsebene heraus.

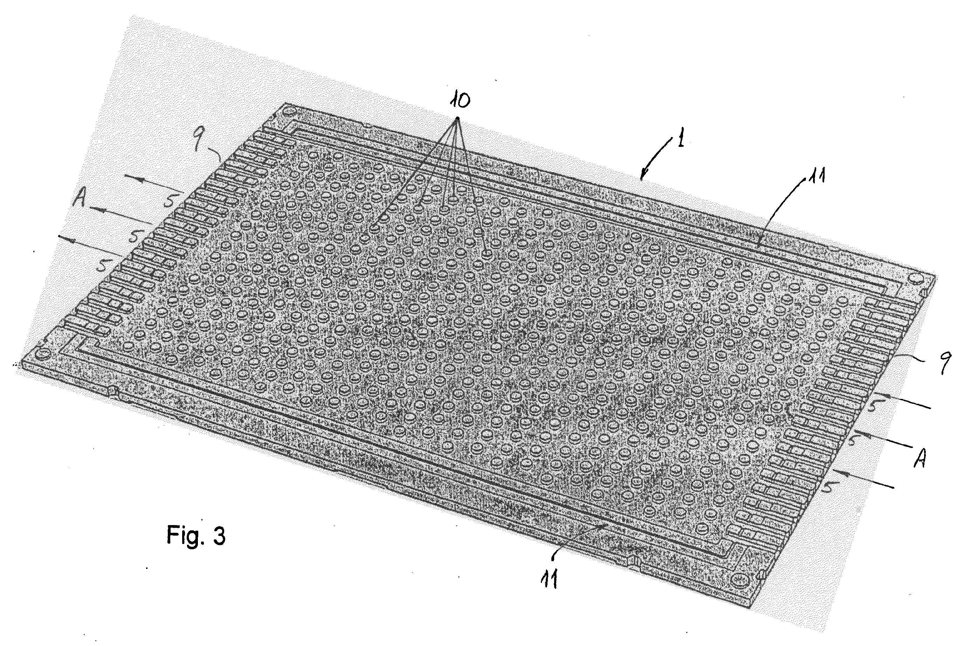

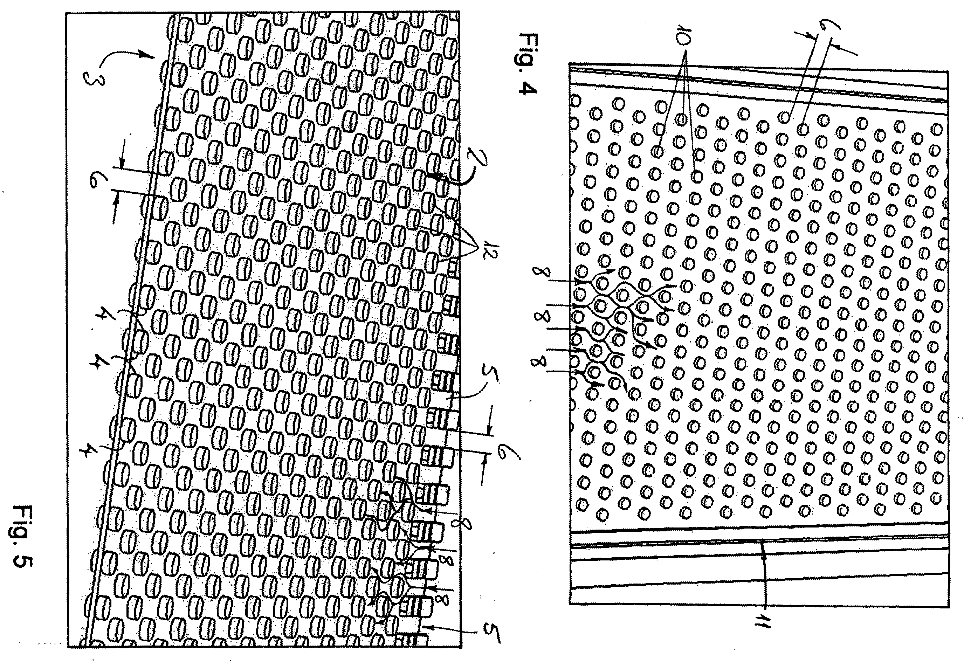

Mit Bezug auf die Figuren 3 bis 5 wird nun eine strukturierte Membran 1 zur Anwendung in einer Gastransfervorrichtung 20 gemäß einem bevorzugten Ausführungsbeispiel der Erfindung beschrieben. Wie in den Figuren 3 bis 5 wieder stark vergrößert gezeigt, ist diese Membran derart strukturiert, dass sie sowohl gerade Kanäle 5 als Verästelungen bzw. verästelte oder verzweigte Wege 8 für den Durchfluss der flüssigen und gasförmigen Phasen bildet. In diesen Figuren sieht man hauptsächlich nur die obere Membranseite 2 aber die untere Seite 3 ist analog gestaltet. An den gegenüber liegenden Enden 9 sind parallele verlaufende, quadratische Aussparungen, die eine Reihe von nebeneinander angeordneten Kanälen 5 z.B. für eine biologische Flüssigkeit bilden. Diese Kanäle 5 gehören der ersten Kammer 21 zu und bilden Einlässe 23 bzw. Auslässe 24 in die erste Kammer 21 mit Flussrichtung gezeigt von den Pfeilen A. Die Kanäle 5 stehen in Verbindung mit einem breiten, zentralen Feld auf der Membranoberseite 2, das mit zylinderförmigen Vorsprüngen bzw. Noppen 10 bestückt ist, die im Wesentlichen senkrecht aus der Membranebene herausragen. Wie aus der Figur 5 zu erahnen ist, sieht die untere Membranseite 3 genauso aus. Die Wände 4 der Kanäle 5 und der Vorsprünge bzw. Noppen 10 stehen alle in einem Abstand 6 von einander im Bereich von ca. 20 bis 150 µm. Ferner sind die Vorsprünge bzw. Noppen 10 derart angeordnet, dass sie als Hindernisse in der Flussrichtung stehen und eine Ablenkung und eine Aufspaltung der Strömung durch die jeweilige Kammer verursachen. Diese Ablenkung und Aufspaltung der Strömung führt wiederum zu Verästelungen 8 in der Kammer oder, anders gesagt, zu verästelten oder verzweigten Durchflusswegen 8, wie mit den Flusspfeilen dargestellt. Da diese Membran 1, wie in Figur 2, von oben und unten mit einem Trägermaterial 7 versehen und stabilisiert wird, entstehen die Hohlräume, die die obere erste Kammer 21 und die untere zweite Kammer 22 schaffen. Das Anbringen und die Verbindung der Trägerschichten 7 erfolgen wie oben beschrieben. Am Rand der oberen (und unteren) Membranseite 2 ist eine rechteckige Aussparung 11 zur Aufnahme der Trägerschicht 7, wenn die Membran 1 von oben mit dieser Schicht 7 gedeckelt wird. Die innere Seite der Trägerschicht 7 kann auch in Kontakt und Verbindung mit den oberen Enden 12 der Vorsprünge bzw. Noppen 10 aber dies ist nicht unbedingt nötig. Die oberen Enden 12 der Vorsprünge bzw. Noppen können auch in der jeweiligen Kammer frei sein.

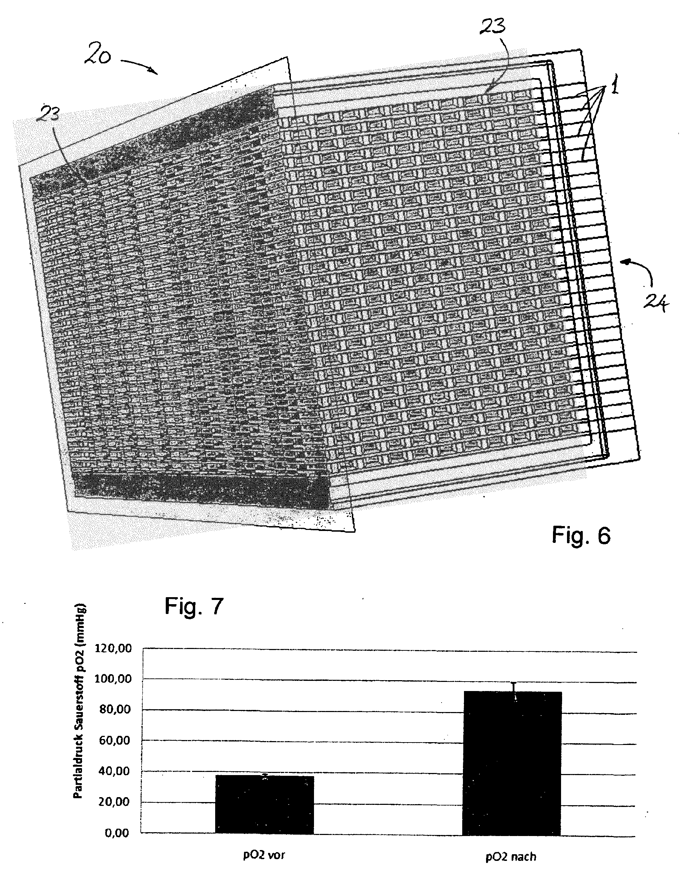

Figur 6 zeigt eine Gastransfervorrichtung 20 gemäß einem bevorzugten Ausführungsbeispiel der Erfindung in einer perspektivischen Ansicht. Die Vorrichtung 20 besteht aus einem Membranstapel. Die Membrane 1 sind ähnlich wie in den Figuren 3 bis 5 gezeigt aber die Einlässe 23 und Auslässe 24 auf der unteren Seite 3 sind rechtwinkelig ausgerichtet zu denen auf der oberen Seite 2, wie in Figur 1. Damit kann z.B. eine biologische Flüssigkeit wie Blut in einer Richtung B über die obere Seite jeder Membran 1 fließen und Sauerstoff O in einer Querrichtung über die untere Seite 3 jeder Membran 1 fließen. Das Übereinander-Stapeln von den einzelnen Membranen 1 ergibt eine quaderförmige Anordnung, welche mit entsprechenden Dichtungen an den Rändern und geeigneten Anschlüssen zum Anschließen von Schläuchen als Modul in einer Gastransfervorrichtung z.B. in einer künstlichen Lunge dienen.

Figur 7 ist eine Graphik, welche die Ergebnisse einer Anreicherung von Sauerstoff in einer Testflüssigkeit in einem einzelnen Mikrokanal zeigt. Der Mikrokanal war mit einer Mischerstruktur (d.h. einem verschlängelten Kanal) versehen und hatte eine Länge von 66 mm und einen quadratischen Querschnitt von 320 µm Tiefe und 320 µm Breite. Die Testflüssigkeit wurde mit einem Volumenstrom von 2,4 ml/min durch den Mikrokanal gepumpt. Die Graphik zeigt eine erhebliche Anreicherung von Sauerstoff (als Partialdruck in der Flüssigkeit) nach dem Verfahren mit der erfindungsgemäßen Gastransfervorrichtung.

Wie vom Fachmann verstanden wird, kann die erfindungsgemäße Gastransfervorrichtung anders als in den Figuren gestaltet werden, ohne von den allgemeinen, in den Ansprüchen angegebenen Merkmalen der Erfindung abzuweichen. Zum Beispiel könnte sich die zweite Kammer in der ersten Kammer befinden bzw. von der ersten Kammer im Wesentlichen umgeben sein. Alternativ könnte sich die erste Kammer in der zweiten Kammer befinden bzw. von der zweiten Kammer im Wesentlichen umgeben sein. Wie vom Fachmann auch verstanden wird, ist die erfindungsgemäße Vorrichtung nicht auf maximale eine Behandlung der biologischen Flüssigkeit begrenzt. Vielmehr lässt der Aufbau der erfindungsgemäßen Vorrichtung eine Mehrzahl von möglichen gleichzeitigen Behandlungen zu.

## Patentansprüche

1. Gastransfervorrichtung, umfassend mindestens zwei Kammern (21, 22) und mindestens eine gasdurchlässige und flüssigkeitsundurchlässige Membran (1), wobei die Kammern durch die Membran(en) (1) voneinander getrennt sind, und wobei die Membran(en) auf mindestens einer Seite (2, 3) strukturiert ist/sind und durch diese Struktur Kanäle (5) und/oder Verästelungen (8), insbesondere verästelte Wege, mit Wänden (4) auf der Membran (1) gebildet werden, wobei sich gegenüberliegende Wände (4) eine Beabstandung von ≤ 500 µm, bevorzugt ≤ 350 µm, und weiter bevorzugt ≤ 150 µm aufweisen, und der Anteil der Membranoberfläche, der Kanäle (5) und/oder Verästelungen (8) mit dieser Beabstandung aufweist, mindestens 50 % der Gesamtoberfläche der Membran (1) ausmacht, **dadurch gekennzeichnet, dass** die Membran (1) auf beiden Seiten (2, 3) strukturiert ist, und auf mindestens einer Seite (2) der Membran Verästelungen (8) aufweist, die durch Noppen (10) erzeugt sind, die derart angeordnet sind, dass sie als Hindernisse in der Flussrichtung stehen und eine Ablenkung und Aufspaltung der Strömung durch die jeweils zwei Kammern verursachen.

2. Vorrichtung gemäß Anspruch 1, wobei die Membran auf einer Seite (2) Verästelungen (8) aufweist und auf der anderen Seite (3) Kanäle (5) aufweist.

3. Vorrichtung gemäß Anspruch 1 oder 2, wobei die mindestens zwei Kammern (21, 22) mindestens teileweise von der Membran bzw. von der Membranstruktur gebildet sind, und/oder
wobei die Wände (4) der Struktur auf der mindestens einen Seite der Membran aus einer Ebene der Membran herausragen bzw. hervorstehen und/oder in eine der Kammern (21, 22) hineinragen bzw. hineinreichen.

4. Vorrichtung gemäß einem der vorhergehenden Ansprüche, wobei die mindestens zwei Kammern (21, 22) eine erste Kammer (21) zur Aufnahme einer biologischen Flüssigkeit und eine zweite Kammer (22) zur Aufnahme eines Gases umfassen, wobei die durch die Struktur gebildeten Kanäle (5) und/oder Verästelungen (8) einem Transfer von Gasmolekülen zwischen den ersten und zweiten Kammern dienen, vorzugsweise wobei die erste Kammer in mehreren Kammern unterteilt ist, sodass die Vorrichtung mehrere erste Kammern umfasst, die von der zweiten Kammer durch eine gasdurchlässige und flüssigkeitsundurchlässige Membran getrennt sind; und/oder
wobei die zweite Kammer in mehreren Kammern unterteilt ist, sodass die Vorrichtung mehrere zweite Kammern umfasst, die von der ersten Kammer durch eine gasdurchlässige und flüssigkeitsundurchlässige Membran getrennt sind, noch mehr bevorzugt wobei die mehreren ersten Kammern in einer oder mehreren Reihen nebeneinander, z.B. in einem Abstand voneinander, und/oder in mehreren Lagen übereinander angeordnet sind,
und/oder wobei die mehreren zweiten Kammern in einer oder mehreren Reihen nebeneinander, z.B. in einem Abstand voneinander, und/oder in mehreren Lagen übereinander angeordnet sind.

5. Vorrichtung gemäß Anspruch 3, wobei eine längliche Ausrichtung der ersten Kammer(n) (21) sich quer und bevorzugt rechtwinkelig zu einer länglichen Ausrichtung der zweiten Kammer(n) (22) erstreckt.

6. Vorrichtung gemäß einem der vorhergehenden Ansprüche, wobei mindestens eine, und bevorzugt jede, der Kammern (21, 22) als Durchflusskammer gestaltet ist, und/oder
wobei die erste Kammer (21) für einen Durchfluss von einem Einlass zu einem Auslass in einer Richtung gegen oder quer zur zweiten Kammer (22) ausgebildet ist, und/oder
wobei die Membran (1) aus einem organischen Material besteht oder ein solches umfasst, vorzugsweise wobei das organische Material ein Polymer, Polymerkomposit oder Polymerschichtung ist, noch mehr bevorzugt wobei das Polymer ein Polyolefin ist, vorzugsweise Polymethylpenten.

7. Vorrichtung gemäß einem der vorhergehenden Ansprüche, wobei die Membran (1) als Membranstapel ausgestaltet ist.

8. Vorrichtung gemäß einem der vorhergehenden Ansprüche, wobei die Membran (1) durch Spritzguss bzw. Mikrospritzguss oder ein Heißprägeverfahren strukturiert ist.

9. Vorrichtung gemäß einem der vorhergehenden Ansprüche, wobei die Membran (1) eine Stärke von 10 bis 100 µm aufweist, vorzugsweise von 20 bis 50 µm.

10. Vorrichtung gemäß einem der vorhergehenden Ansprüche, wobei die Membran (1) selektiv im Wesentlichen für Sauerstoff und/oder Kohlenstoffdioxid durchlässig ist, und/oder
wobei die Membran (1) nicht oder nur geringfügig für Stickstoff durchlässig ist.

11. Vorrichtung gemäß einem der vorhergehenden Ansprüche, wobei die Membran (1) durch ein Trägermaterial stabilisiert ist, und/oder
wobei die Membran (1) mit Zellen, vorzugsweise Epithelzellen, besiedelt ist, und/oder
wobei die Membran (1) mit einer oder mehreren Substanzen, ausgewählt aus der Gruppe, bestehend aus (Poly)Saccharid, vorzugsweise Heparin, Nukleinsäure, Protein, vorzugsweise Albumin, beschichtet ist.

12. Vorrichtung gemäß einem der vorhergehenden Ansprüche, wobei eine Kammer (21, 22) einen Durchmesser von 1 µm bis 1 cm aufweist, und/oder
wobei eine zweite oder weitere Kammer (21, 22) einen Durchmesser von 1 µm bis 1 cm aufweist.

13. Vorrichtung gemäß einem der vorhergehenden Ansprüche, wobei die biologische Flüssigkeit ausgewählt ist aus der Gruppe, bestehend aus Blut, Blutserum, Zellsuspension, Zelllösung und Kulturmedium.

14. Vorrichtung, bestehend aus zwei oder mehreren der Vorrichtungen gemäß einem der vorhergehenden Ansprüche.

## Claims

1. A gas transfer device comprising at least two chambers (21, 22) and at least one gas-permeable and liquid-impermeable membrane (1), wherein the chambers are separated from one another by the membrane(s) (1), and wherein the membrane(s) is/are structured on at least one side (2, 3) and channels (5) and/or branching structures (8), in particular branched pathways, formed by walls (4) on the membrane (1), wherein the walls (4) which are opposite to each other have a spacing of ≤ 500 µm, preferably of ≤ 350 µm, and more preferably of ≤ 150 µm, and the proportion of the membrane surface area which comprises channels (5) and/or branching structures (8) having this spacing constitutes at least 50% of the total surface area of the membrane (1),
**characterized in that** the membrane (1) is structured on both sides (2, 3) and is provided on at least one side (2) of the membrane with branching structures, that are generated by bumps (10) which are arranged such that they stand as obstructions in the direction of flow and cause deflection and splitting of flow through the respective chamber.

2. A device according to claim 1, wherein the membrane is provided on one side (2) with branching structures (8) and is provided on the other side (3) with channels (5).

3. A device according to claim 1 or 2, wherein the at least two chambers (21, 22) are formed at least in part by the membrane or by the membrane structure and/or wherein the walls (4) of the structure on the at least one side of the membrane project or stand out from a plane of the membrane and/or project or extend into one of the chambers (21, 22).

4. A device according to any one of the preceding claims, wherein the at least two chambers (21, 22) comprise a first chamber (21) to receive a biological liquid and a second chamber (22) to receive a gas, wherein the channels (5) and/or branching structures (8) formed by the structure serve for the transfer of gas molecules between the first and second chambers, and/or
wherein the first chamber is subdivided into a plurality of chambers, such that the device comprises a plurality of first chambers, which are separated from the second chamber by a gas-permeable and liquid-impermeable membrane; and/or
wherein the second chamber is subdivided into a plurality of chambers, such that the device comprises a plurality of second chambers which are separated from the first chamber by a gas-permeable and liquid-impermeable membrane, and/or
wherein the plurality of first chambers are arranged next to one another in one or more rows, for example spaced apart from one another, and/or one above the other in a plurality of layers.

5. A device according to claim 3, wherein a lengthwise orientation of the first chamber(s) (21) extends crosswise and preferably at right angles to a lengthwise orientation of the second chamber(s) (22).

6. A device according to any one of the preceding claims, wherein at least one, and preferably each one, of the chambers (21, 22) is constructed as a through-flow chamber, and/or
wherein the first chamber (21) is constructed for through-flow from an inlet to an outlet in a direction contrary or crosswise to the second chamber (22),
wherein the membrane (1) consists of or comprises an organic material, wherein the organic material preferably is a polymer, polymer composite or polymer multilayer, wherein the polymer is even more preferably a polyolefin, preferably polymethylpentene.

7. A device according to any one of the preceding claims, wherein the membrane (1) takes the form of a membrane stack.

8. A device according to any one of the preceding claims, wherein the membrane (1) is structured by injection moulding or micro-injection moulding or a hot stamping method.

9. A device according to any one of the preceding claims, wherein the membrane (1) has a thickness of 10 to 100 µm, preferably of 20 to 50 µm.

10. A device according to any one of the preceding claims, wherein the membrane (1) is substantially selectively permeable to oxygen and/or carbon dioxide and/or wherein the membrane (1) is impermeable or only slightly permeable to nitrogen.

11. A device according to any one of the preceding claims, wherein the membrane (1) is strengthened by a support material, and/or wherein the membrane (1) is colonised with cells, preferably epithelial cells, and/or
wherein the membrane (1) is coated with one or more substances selected from the group consisting of (poly)saccharide, preferably heparin, nucleic acid, protein, preferably albumin.

12. A device according to any one of the preceding claims, wherein a chamber (21, 22) has a diameter of 1 µm to 1 cm, and/or wherein a second or further chamber has a diameter of 1 µm to 1 cm.

13. A device according to any one of the preceding claims, wherein the biological liquid is selected from the group consisting of blood, blood serum, cell suspension, cell solution and culture medium.

14. A device consisting of two or more of the devices according to any one of the preceding claims.

## Revendications

1. Dispositif de transfert de gaz, comprenant au moins deux chambres (21, 22) et au moins une membrane (1) perméable aux gaz et imperméable aux liquides, les chambres étant séparées les unes des autres par la ou les membranes (1), et la ou les membranes étant structurées sur au moins un côté (2, 3) et des canaux (5) et/ou des ramifications (8), en particulier des chemins ramifiés, étant formés avec des parois (4) sur la membrane (1) du fait de ladite structure, sachant que les parois (4) qui se font face présentent un espacement de ≤ 500 µm, de manière préférée ≤ 350 µm, de manière davantage préférée de ≤ 150 µm, et la proportion de la surface de membrane, qui présente des canaux (5) et/ou des ramifications (8) présentant ledit espacement, représente au moins 50 % de la surface totale de la membrane (1),
**caractérisé en ce que** la membrane (1) est structurée sur deux côtés (2, 3) et présente, sur au moins un côté (2) de la membrane, des ramifications (8) qui sont produites par des boutons (10), qui sont disposés de telle manière qu'ils forment des obstacles se dressant dans la direction d'écoulement et provoquent une déviation et une scission de l'écoulement dans la chambre correspondante, respectivement.

2. Dispositif selon la revendication 1, dans lequel la membrane présente, sur un côté (2), des ramifications (8) et, sur l'autre côté (3), des canaux (5).

3. Dispositif selon la revendication 1 ou 2, les deux chambres (21, 22) ou plus étant formées au moins en partie par la membrane ou par la structure de membrane, et/ou les parois (4) de la structure sur l'au moins un côté de la membrane dépassant en sortant ou faisant saillie d'un plan de la membrane et/ou dépassant ou pénétrant à l'intérieur d'une des chambres (21, 22).

4. Dispositif selon l'une quelconque des revendications précédentes, les deux chambres (21, 22) ou plus comprenant une première chambre (21) servant à recevoir un liquide biologique et une deuxième chambre (22) servant à recevoir un gaz, les canaux (5) et/ou ramifications (8) formés par la structure servant à un transfert de molécules de gaz entre les premières et deuxièmes chambres, la première chambre étant divisée de préférence en plusieurs chambres de sorte que le dispositif comprend plusieurs premières chambres, qui sont séparées de la deuxième chambre par une membrane perméable aux gaz et imperméable aux liquides ;
et/ou
la deuxième chambre étant divisée en plusieurs chambres de sorte que le dispositif comprend plusieurs deuxièmes chambres, qui sont séparées de la première chambre par une membrane perméable aux gaz et imperméable aux liquides, voire les nombreuses premières chambres étant disposées plus préférentiellement dans une ou plusieurs rangées les unes à côté des autres, par exemple à une distance donnée les unes des autres, et/ou en plusieurs couches les unes sur les autres,
les nombreuses deuxièmes chambres étant disposées dans une ou plusieurs rangées les unes à côté des autres, par exemple à une distance donnée les unes des autres, et/ou en plusieurs couches les unes sur les autres.

5. Dispositif selon la revendication 3, une orientation longitudinale de la/des première(s) chambre(s) (21) s'étendant de manière transversale et de préférence à angle droit par rapport à une orientation longitudinale de la/des deuxièmes chambres (22).

6. Dispositif selon l'une quelconque des revendications précédentes, dans lequel au moins une des chambres (21, 22), et de manière préférée chaque chambre, est configurée sous la forme d'une chambre de passage de flux, et/ou
la première chambre (21) est réalisée pour un passage de flux allant d'une entrée vers une sortie dans une direction à l'opposé ou de manière transversale par rapport à la deuxième chambre (22), et/ou
la membrane (1) est constituée d'un matériau organique ou comprenant un matériau de ce type, le matériau organique étant de préférence un polymère, un composite polymère ou un empilement de couches polymères, voire le polymère étant plus préférentiellement une polyoléfine, de préférence un polyméthylpentène.

7. Dispositif selon l'une quelconque des revendications précédentes, dans lequel la membrane (1) est configurée sous la forme d'un empilement de membranes.

8. Dispositif selon l'une quelconque des revendications précédentes, dans lequel la membrane (1) est structurée par un moulage par injection ou un moulage par micro-injection ou par un procédé d'estampage à chaud.

9. Dispositif selon l'une quelconque des revendications précédentes, la membrane (1) présentant une épaisseur allant de 10 à 100 µm, de préférence allant de 20 à 50 µm.

10. Dispositif selon l'une quelconque des revendications précédentes, la membrane (1) étant perméable au choix essentiellement à l'oxygène et/ou au dioxyde de carbone, et/ou
la membrane (1) n'étant pas perméable ou seulement légèrement perméable à l'azote.

11. Dispositif selon l'une quelconque des revendications précédentes, dans lequel la membrane (1) est stabilisée par un matériau de support, et/ou
la membrane (1) est colonisée par des cellules, de préférence des cellules épithéliales, et/ou
la membrane (1) est revêtue d'une ou de plusieurs substances choisies parmi le groupe constitué d'un (poly)saccharide, de préférence de l'héparine, d'un acide nucléique, d'une protéine, de préférence de l'albumine.

12. Dispositif selon l'une quelconque des revendications précédentes, dans lequel une chambre (21, 22) présente un diamètre allant de 1 µm à 1 cm,
et/ou
une deuxième ou une autre chambre (21, 22) présente un diamètre allant de 1 µm à 1 cm.

13. Dispositif selon l'une quelconque des revendications précédentes, dans lequel le liquide biologique est choisi parmi le groupe constitué du sang, du sérum sanguin, d'une suspension cellulaire, d'une solution cellulaire et d'un milieu de culture.

14. Dispositif constitué de deux ou de plusieurs des dispositifs selon l'une quelconque des revendications précédentes.
